(19) Europäisches Patentamt
European Patent Office
Office européen des brevets

(11) EP 3 202 493 B1

# (12) EUROPÄISCHE PATENTSCHRIFT

(45) Veröffentlichungstag und Bekanntmachung des Hinweises auf die Patenterteilung:
**03.04.2019 Patentblatt 2019/14**

(51) Int Cl.:
***B01J 25/00*** *(2006.01)* ***B01J 37/00*** *(2006.01)*
***C07C 209/48*** *(2006.01)* ***C07C 211/09*** *(2006.01)*

(21) Anmeldenummer: **16154364.0**

(22) Anmeldetag: **05.02.2016**

(54) **VERFAHREN ZUR HERSTELLUNG VON TRIMETHYLHEXAMETHYLENDIAMIN**

METHOD FOR THE PREPARATION OF TRIMETHYLHEXAMETHYLENE DIAMINE

PROCÉDÉ DE FABRICATION DE TRIMÉTHYLE-HEXAMÉTHYLÈNE-DIAMINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.08.2017 Patentblatt 2017/32**

(73) Patentinhaber: **Evonik Degussa GmbH**
**45128 Essen (DE)**

(72) Erfinder:
- **RITTSTEIGER, Anne**
**59399 Olfen (DE)**
- **RÜFER, Alexander Martin**
**45657 Recklinghausen (DE)**
- **SCHNEIDER, Sven**
**45711 Datteln (DE)**
- **RÖDER, Stefan**
**36391 Sinntal (DE)**
- **BERWEILER, Monika**
**63477 Maintal (DE)**

(56) Entgegenhaltungen:
**EP-A1- 2 114 859** **DE-A1- 2 159 736**
**GB-A- 611 987**

EP 3 202 493 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein verbessertes Verfahren zur Herstellung von Trimethylhexamethylendiamin, nachfolgend abgekürzt TMD genannt, durch Hydrierung von Trimethylhexamethylendinitril, nachfolgend abgekürzt TMN genannt, in Gegenwart eines heterogenen Katalysators.

TMD findet Verwendung als Epoxidharzhärter, als Aminkomponente in Polyamiden sowie als Ausgangskomponente für Trimethylhexamethylendiisocyanat, das seinerseits wiederum Ausgangskomponente für Polyurethansysteme ist.

TMD wird industriell durch Hydrierung von TMN hergestellt:

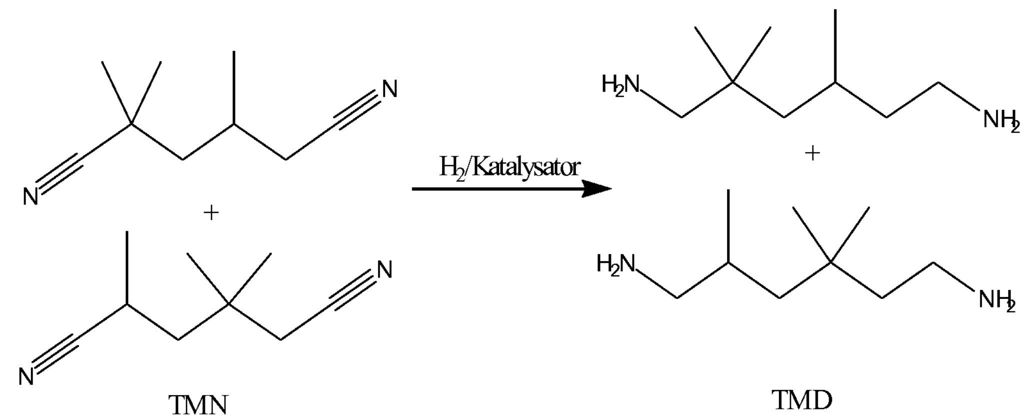


TMN TMD

Bedingt durch den Herstellungsprozess des TMN wird in der Hydrierung ein Gemisch aus 2,4,4-Trimethylhexamethylendinitril und 2,2,4-Trimethylhexamethylendinitril eingesetzt (Verhältnis ca. 60:40). Durch Hydrierung wird ein entsprechendes Isomerengemisch aus 2,4,4-Trimethylhexamethylendiamin und 2,2,4-Trimethylhexamethylendiamin erhalten.

Aktivierte Metallkatalysatoren sind in der chemischen Technik als Raney-Typ Katalysatoren bekannt. Sie werden überwiegend als Pulverkatalysatoren bei einer Großzahl von Hydrierreaktionen eingesetzt. Raney-Typ Katalysatoren werden aus einer Legierung bestehend aus mindestens einer katalytisch aktiven Metallkomponente und mindestens einer auslaugbaren Legierungskomponente hergestellt. Als katalytisch aktive Komponente kommen hauptsächlich Nickel, Kobalt, Kupfer und Eisen zum Einsatz. Als auslaugbarer Legierungsbestandteil findet überwiegend Aluminium Verwendung, aber auch Zink und Silizium sind geeignet. Die so genannte Raney-Legierung wird üblicherweise fein vermahlen, und anschließend wird die auslaugbare Komponente durch Auslaugen mit Alkalien, wie z.B. Natronlauge, ganz oder teilweise entfernt.

Pulverkatalysatoren haben den Nachteil, dass sie nur in Batch-Verfahren eingesetzt werden können. Es wurden daher bereits verschiedene Verfahren beschrieben, die die Herstellung von aktivierten Metall-Festbettkatalysatoren ermöglichen. Solche Festbettkatalysatoren nach Raney sind für die großtechnische Herstellung von TMD besonders geeignet, da sie eine kontinuierliche Prozessführung ermöglichen.

In der DE 43 45 265 und DE 43 35 360 werden geformte Raney-Katalysatoren auf Basis von Ni, Co, Cu und Fe beschrieben, die zur Hydrierung organischer Verbindungen geeignet sind. Der Nachteil der Katalysatoren liegt darin, dass den Katalysatoren Metallpulver als Binder zugesetzt werden muss, welches im Vergleich zum katalytisch aktiven Metall eine geringere Aktivität aufweist.

In der EP 880 996 wird die Herstellung geformter Raney-Katalysatoren beschrieben, die ohne Zusatz von metallischen Bindemitteln hergestellt werden und zur Hydrierung von Nitrilen eingesetzt werden können. Zur Herstellung dieser Katalysatoren wird eine als Pulver vorliegende Metall-Aluminium-Legierung mit einem hochmolekularen Polymer sowie ggf. Promotoren vermischt und anschließend zu Formkörpern geformt, z.B. durch Extrusion. Die Formkörper werden anschließend bei Temperaturen von bis zu 850°C kalziniert. Die Temperaturbehandlung führt zur kontrollierten Zersetzung des Polymeren und zur Ausbildung eines Festbettkatalysators mit ausreichender mechanischer Stabilität. Anschließend erfolgt die Aktivierung durch teilweise oder vollständige Auslaugung des Aluminiums mittels Natronlauge.

Aus der EP 2 114 859 ist ein Verfahren zur Herstellung von TMD aus TMN bekannt, bei dem ein Formkörper als Raney-Typ-Katalysator für die Hydrierung verwendet wird. Dieser Katalysator wird durch das Aufsprühen einer Suspension bestehend aus mindestens dem Legierungspulver und mindestens einem Lösungsmittel auf ein Trägermaterial hergestellt. Optional können noch Binder und Promotoren der Suspension zugegeben werden. Nachteil ist die aufwändige Herstellung des Katalysators über ein mehrstufiges Verfahren bestehend aus Bereitstellung und Aufsprühen der Legierungssuspension auf ein Trägermaterial, Trocknen, Kalzinieren und Aktivieren. Ein weiterer Nachteil ist die

mechanische Stabilität bzw. Haftung der katalytisch aktiven Schicht auf der Oberfläche des Trägermaterials.

Aufgabe der vorliegenden Erfindung ist es, ein Verfahren zur Herstellung von TMD aus TMN zu entwickeln, bei dem Hydrierkatalysatoren nach Raney eingesetzt werden, die über ein einfacheres Verfahren als Festbettkatalysatoren hergestellt werden können und trotzdem gleiche oder bessere TMD-Selektivitäten erzielt werden als mit den bisher bekannten Verfahren, in denen Hydrierkatalysatoren nach Raney eingesetzt werden.

Überraschend wurde nun gefunden, dass eine Hydrierung von TMN zu TMD an einem heterogenen Katalysator mit einer sehr hohen Selektivität möglich ist.

Gegenstand der Erfindung ist ein Verfahren zur Herstellung von Trimethylhexamethylendiamin durch Hydrierung von Trimethylhexamethylendinitril enthaltenden Gemischen in Gegenwart von mindestens Ammoniak und Wasserstoff und eines Katalysators, in An- oder Abwesenheit von Lösungsmittel,

wobei der Katalysator die folgenden Eigenschaften aufweist:

I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

und

II. Der Katalysator liegt in Form von unregelmäßigen Partikeln als Granulat vor und weist nach der Aktivierung Partikelgrößen von 1 bis 8 Millimeter (mm) auf.

Katalysator

Der Katalysator besteht aus einer Metall-Legierung, wobei die Metall-Legierung durch Basen auf der Oberfläche aktiviert ist. Hierfür wird Aluminium teilweise oder vollständig aus der Legierung ausgelaugt. Die Schichtdicke der aktivierten Schicht auf der Partikeloberfläche des Katalysators beträgt bevorzugt 50 bis 1000 Mikrometer ($\mu$m). Sie kann aber auch größer oder kleiner sein. Auf der Oberfläche befindet sich demnach die katalytisch aktive Zusammensetzung des Katalysators.

Der erfindungsgemäße Katalysator liegt nach der Aktivierung als Granulat in Form einzelner Partikel vor.

Der erfindungsgemäße Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

1. Variante

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

oder

2. Variante

| | |
|---|---|
| Cobalt: | 55 bis 90 Gew.-% |
| Aluminium: | 5 bis 44,5 Gew.-% |
| Chrom: | 0,5 bis 5 Gew.-% |

oder

3. Variante

| | |
|---|---|
| Cobalt: | 55 bis 88 Gew.-% |
| Aluminium: | 5 bis 44,5 Gew.-% |
| Nickel: | 0,5 bis 7 Gew.-% |

oder
4. Variante

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

5. Variante

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

Gesamtheit bedeutet, dass bei der Zusammensetzung nicht zwischen dem Gehalt der Metalle auf der Oberfläche und in der aktivierten Schicht und im Kern der Katalysator-Partikel unterschieden wird, sondern Alles zusammen addiert und berechnet wird.

Der Katalysator liegt in Form von unregelmäßigen Partikeln also als Granulat vor.

Zusätzlich weist der erfindungsgemäße Katalysator nach der Aktivierung die folgenden Partikelgrößen auf:
Im Allgemeinen kann der Katalysator, also die Granulat-Teilchen Partikelgrößen von 1 bis 8 Millimeter (mm) aufweisen.

In einer ersten bevorzugten Variante der Erfindung variieren die Partikelgrößen des Katalysators, also die Granulat-Teilchen von 2,5 bis 6 Millimeter (mm).

In einer zweiten bevorzugten Variante der Erfindung variieren die Partikelgrößen des Katalysators, also die Granulat-Teilchen von 3 bis 7 Millimeter (mm).

In einer dritten bevorzugten Variante der Erfindung variieren die Partikelgrößen des Katalysators, also die Granulat-Teilchen von 2 bis 5 Millimeter (mm).

Die angegebenen Partikelgrößen können auch innerhalb der Bereiche eine statistische Verteilung der Größe aufweisen. Dabei sind enge Verteilungen als auch breite Verteilungen erfindungsgemäß.

Die Bestimmung der Partikelgrößen wird in der DIN ISO 9276-1 (September 2004) und 9276-2 (Februar 2006) und 9276-4 (Februar 2006) und 9276-6 (Januar 2012) beschrieben. Außerdem findet man genaue Angaben zur Definition von Partikelgrößen, der Verteilung von Partikelgrößen und der Messung von Partikelgrößen in HORIBA® Scientific, A GUIDEBOOK TO PARTICLE SIZE ANALYSIS, 2012, von HORIBA® Instruments, Inc, Irvine, USA.

Erfindungsgemäß kann die Verteilung der Partikelgrößen und die Messung der Partikelgrößen durch Laserverfahren (ISO 13320, 2012), Lichtverfahren oder Bildverfahren bestimmt werden.

Bevorzugt wird der erfindungsgemäße Katalysator durch Siebung der hergestellten Granulate erhalten. Dabei werden sogenannte Siebfraktionen hergestellt. Es können dabei einzelne Siebfraktionen gemischt werden, oder es wird ein Katalysator durch einmalige oder mehrmalige Siebung erhalten. Die so hergestellten Katalysatoren weisen bei den Partikelgrößen eine statistische Verteilung auf, üblicherweise in Form einer Gauß-Verteilung. Es sind symmetrische aber auch asymmetrische Verteilungen möglich.

In einer vierten bevorzugten Variante der Erfindung variieren die Partikelgrößen des Katalysators, also die Granulat-Teilchen mit einer statistischen Verteilung zwischen 2,5 bis 5,5 Millimeter (mm), und wobei bis zu 10 % der Partikel oberhalb der genannten Obergrenze und bis zu 10 % der Partikel unterhalb der genannten Untergrenze liegen können.

In einer fünften bevorzugten Variante der Erfindung variieren die Partikelgrößen des Katalysators, also die Granulat-Teilchen mit einer statistischen Verteilung zwischen 3,5 bis 6,5 Millimeter (mm), und wobei bis zu 10 % der Partikel oberhalb der genannten Obergrenze und bis zu 10 % der Partikel unterhalb der genannten Untergrenze liegen können.

In einer sechsten bevorzugten Variante der Erfindung variieren die Partikelgrößen des Katalysators, also die Granulat-Teilchen mit einer statistischen Verteilung zwischen 2 bis 5 Millimeter (mm), und wobei bis zu 10 % der Partikel oberhalb der genannten Obergrenze und bis zu 10 % der Partikel unterhalb der genannten Untergrenze liegen können.

In einer siebten bevorzugten Variante der Erfindung variieren die Partikelgrößen des Katalysators, also de Granulat-Teilchen mit einer statistischen Verteilung zwischen 3 bis 7 Millimeter (mm), und wobei bis zu 10 % der Partikel oberhalb der genannten Obergrenze und bis zu 10 % der Partikel unterhalb der genannten Untergrenze liegen können.

Geeignete Methoden und Beschreibungen zur Siebanalyse sind beschrieben in: DIN 66165-1:1987-04 Partikelgrößenanalyse; Siebanalyse; Grundlagen, und in DIN 66165-2:1987-04 Partikelgrößenanalyse; Siebanalyse; Durchführung.

Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung.

Jörg Hoffmann: Handbuch der Messtechnik. Hanser Verlag, 2007, ISBN 978-3-446-40750-3, Kapitel 3.12.16.2.1.

Der erfindungsgemäße Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und mit

Partikelgrößen des Katalysators, also die Granulat-Teilchen mit einer statistischen Verteilung zwischen 2,5 bis 5,5 Millimeter (mm),

oder

Partikelgrößen des Katalysators, also die Granulat-Teilchen mit einer statistischen Verteilung zwischen 3,5 bis 6,5 Millimeter (mm),

oder

Partikelgrößen des Katalysators, also die Granulat-Teilchen mit einer statistischen Verteilung zwischen 2 bis 5 Millimeter (mm),

oder

Partikelgrößen des Katalysators, also die Granulat-Teilchen mit einer statistischen Verteilung zwischen 3 bis 7 Millimeter (mm),

und wobei bis zu 10 % der Partikel oberhalb der genannten Obergrenze und bis zu 10 % der Partikel unterhalb der genannten Untergrenze liegen können.

Allgemeine Methode zur Herstellung des Katalysators:

a) Herstellung der Legierung

Die Herstellung der Legierung erfolgt thermisch, zum Beispiel in einem Induktionsofen. Es werden dabei die Metalle geschmolzen und eine Legierung erhalten. Die fertige Schmelze wird für die Weiterverarbeitung zum Beispiel zu Barren gegossen.

b) Herstellung der Granulate

Die Legierung wird in geeigneten Geräten zu Granulaten verarbeitet, zum Beispiel über einen Backenbrecher vorzerkleinert und über eine Walzenmühle weiter gemahlen. Durch einen Siebschritt wird die gewünschte Größenverteilung der Granulate durch die Wahl der entsprechenden Siebe erhalten (z.B. 3 bis 7 mm).

c) Aktivierung des Katalysators

Die Aktivierung des Katalysators erfolgt in geeigneten Apparaturen. Es können dabei organische oder anorganische Basen eingesetzt werden. Bevorzugt wird eine Lauge (z.B. Natronlauge), verwendet, wobei durch einen exothermen Vorgang ein Teil des Aluminiums unter Bildung von Wasserstoff und Aluminatlauge aus der Legierung herausgelöst wird. Die Konzentration der Lauge kann zwischen 5 und 30 Gew.-% liegen und die Reaktionstemperatur zwischen 50 und 120°C. Der Grad der Aktivierung wird über die Temperatur und die Reaktionszeit bestimmt. Die benötigte Reaktionszeit für einen bestimmten Aktivierungsgrad ist dabei direkt abhängig von den gewählten Reaktionsbedingungen. Nach der Aktivierung wird der Katalysator mit Wasser gewaschen und anschließend unter Wasser gelagert.

Andere Zusammensetzungen können im Herstellungsschritt a) durch die entsprechende Wahl der Metallmengen analog produziert werden.

Bevorzugt wird der Katalysator in der beschriebenen Reihenfolge hergestellt. Die Aktivierung des Katalysators kann aber auch vor der Herstellung der Granulate erfolgen.

Zur Erhöhung der Aktivität, Selektivität und/oder Standzeit können die Katalysatoren zusätzlich Dotiermetalle oder andere Modifizierungsmittel enthalten. Typische Dotiermetalle sind z.B. Mo, Fe, Ag, V, Ga, In, Bi, Ti, Zr und Mn

sowie die seltenen Erden, allein oder in Mischungen. Typische Modifizierungsmittel sind z.B. solche, mit denen die Säure-Base-Eigenschaften der Katalysatoren beeinflusst werden können, bevorzugt Alkali- und Erdalkalimetalle bzw. deren Verbindungen, bevorzugt Mg- und Li-Verbindungen. Für den Fall, dass derartige Verbindungen enthalten sind, in einer Menge von maximal ca. 5 Gew.-%, reduziert sich der Anteil der oben genannten Metall Co und Al sowie gegebenenfalls Cr und Ni im Katalysator entsprechend, wobei die Anteile an Co und Al sowie gegebenenfalls Cr und Ni dann nach der Aktivierung sich zu mindestens 95 Gew.-% addieren, bezogen auf die enthaltenden Metalle.

Das erfindungsgemäße Verfahren zur Herstellung von TMD kann batchweise oder kontinuierlich durchgeführt werden. Bevorzugt wird die Hydrierung kontinuierlich in Festbettreaktoren durchgeführt, die in Riesel- oder Sumpffahrweise betrieben werden können. Geeignete Reaktortypen sind z.B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Die Hydrierung erfolgt üblicherweise bei Temperaturen zwischen 20 und 150°C, bevorzugt zwischen 40 und 130°C, und Drücken von 0,3 bis 50 MPa, bevorzugt 5 bis 30 MPa.

Zur Steuerung des Temperaturverlaufs im Reaktor und insbesondere zur Begrenzung der maximalen Temperatur sind verschiedene, dem Fachmann bekannte Methoden, geeignet. So kann z.B. vollständig ohne zusätzliche Reaktorkühlung gearbeitet werden, wobei das Reaktionsmedium die freiwerdende Energie vollständig aufnimmt und dadurch konvektiv aus dem Reaktor heraus führt. Geeignet sind weiterhin zum Beispiel Hordenreaktoren mit Zwischenkühlung, die Verwendung von Wasserstoffkreisläufen mit Gaskühlung, die Rückführung eines Teils des gekühlten Produktes (Kreislaufreaktor) und die Verwendung von externen Kühlmittelkreisläufen, insbesondere bei Rohrbündelreaktoren.

Der für die Hydrierung erforderliche Wasserstoff kann dem Reaktor entweder im Überschuss, beispielsweise mit bis zu 10.000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung von TMN zu TMD an den erfindungsgemäß einzusetzenden Katalysatoren in flüssigem Ammoniak als Lösungsmittel. Pro Mol TMN werden zwischen 1 und 500 Mol, bevorzugt 5 und 200 Mol, besonders bevorzugt zwischen 5 und 100 Mol Ammoniak verwendet.

Obwohl die Hydrierung von TMN zu TMD in Gegenwart von Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol, sowie Ether, besonders THF, MTBE und Dioxan. Der wesentliche Vorteil bei Verwendung eines zusätzlichen Lösungsmittels oder von Lösungsmittelgemischen liegt darin, dass die Hydrierung bei niedrigeren Drücken durchgeführt werden kann, als wenn Ammoniak als alleiniges Lösungsmittel eingesetzt wird.

Das erforderliche Volumen der erfindungsgemäß einzusetzenden Katalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten TMN zu gewährleisten. Üblicherweise liegt der LHSV-Wert bei der Verwendung des bevorzugt einzusetzenden Gemisches aus TMN, Ammoniak und Wasserstoff zwischen 0,5 und 4 $m^3$ TMN-Ammoniak-Mischung pro $m^3$ Katalysator und Stunde, bevorzugt zwischen 1 und 3 $m^3/(m^3 * h)$.

Das den Hydrierreaktor verlassende Reaktionsgemisch wird in an sich bekannter Weise aufgearbeitet. Diese Aufarbeitung umfasst üblicherweise Abtrennen des Ammoniaks, der Lösungsmittel oder Gemische aus Lösungsmittel und Ammoniak, falls Lösungsmittel vorhanden sind, sowie Isolation des TMD. Das abgetrennte Ammoniak und gegebenenfalls weitere abgetrennte Lösungsmittel werden vollständig oder optional nach Ausschleusen eines Teilstroms in den Prozess zurückgeführt.

Das die Hydrierung verlassende Reaktionsgemisch wird mit den üblichen Methoden weiter aufgereinigt, um ein TMD mit der gewünschten Qualität zu erhalten. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z.B. Dephlegmatoren, Trennwänden, ungeordnete Einbauten oder Schüttungen, geordnete Einbauten oder Packungen, Böden mit oder ohne Zwangsführung verwenden.

Außer den zuvor genannten Bestandteilen kann das dem Hydrierreaktor zuzuführende Gemisch zusätzlich höher oder niedriger als TMD siedende Fraktionen enthalten, die bei der destillativen Aufarbeitung des Reaktionsgemisches erhalten werden. Derartige Fraktionen können außer Resten an TMD auch solche Nebenprodukte enthalten, aus denen sich unter Reaktionsbedingungen erneut TMD bildet. Besonders vorteilhaft ist es, nicht vollständig umgesetztes TMN oder Aminonitril enthaltende Fraktionen zurückzuführen.

Es ist bevorzugt aber nicht zwingend erforderlich, dass die erfindungsgemäß einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehrerer Lösungsmittel in Kontakt gebracht.

Unabhängig davon, ob das erfindungsgemäße Verfahren in einer bevorzugten Ausführungsform durchgeführt wird oder nicht, können bei der Umsetzung eines Gemisches aus TMN, Ammoniak, Wasserstoff und gegebenenfalls Lösungsmittel noch eine oder mehrere Hydroxidbasen zugegeben werden. Die Zugabe von Hydroxidbasen kann die Ausbeute an TMD durch Verminderung der Nebenproduktbildung erhöhen. Geeignete Hydroxidbasen sind beispielsweise Alkalihydroxide oder Erdalkalihydroxide. Besonders bevorzugte Hydroxidbasen sind quaternäre Ammoniumhydroxide der allgemeinen Formel ($R^1R^2R^3R^4N$)OH, wobei $R^1$ bis $R^4$ gleich oder unterschiedlich sein können und für aliphatische, cycloaliphatische oder aromatische Reste stehen. Beispiele sind Tetramethyl-, Tetraethyl-, Tetra-n-propyl- und Tetra-n-butylammoniumhydroxid. Geeignete Konzentrationen sind 0,01 bis 100 mmol, bevorzugt 0,05 bis 20 mmol eines Tetraalkylammoniumhydroxids pro Mol TMN.

Es ist auch möglich, bei dem erfindungsgemäßen Verfahren einen oder mehrere Co-Katalysatoren zu verwenden. Geeignete Co-Katalysatoren sind Salze von Kobalt, Nickel, Lanthan, Cer oder Yttrium, bevorzugt Salze von Kobalt und Nickel.

Gegenstand der Erfindung ist auch die Verwendung eines Katalysators zur Herstellung von Trimethylhexamethylendiamin durch Hydrierung von Trimethylhexamethylendinitril enthaltenden Gemischen in Gegenwart von mindestens Ammoniak und Wasserstoff,
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

oder

| | |
|---|---|
| Cobalt: | 55 bis 90 Gew.-% |
| Aluminium: | 5 bis 44,5 Gew.-% |
| Chrom: | 0,5 bis 5 Gew.-% |

oder

| | |
|---|---|
| Cobalt: | 55 bis 88 Gew.-% |
| Aluminium: | 5 bis 44,5 Gew.-% |
| Nickel: | 0,5 bis 7 Gew.-% |

oder

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

oder

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und
II. Der Katalysator liegt in Form von unregelmäßigen Partikeln als Granulat vor und weist nach der Aktivierung Partikelgrößen von 1 bis 8 Millimeter (mm) auf.

Beispiele

Beispiel 1: Herstellung des erfindungsgemäßen Katalysators

a) Herstellung der Legierung

Die Herstellung der Legierung erfolgte in einem Induktionsofen. Es wurden dabei die Metalle in den entsprechenden Mengen bei 1500°C geschmolzen. Die fertige Schmelze wurde für die Weiterverarbeitung zu Barren gegossen.

b) Herstellung der Granulate

Die Legierungsbarren wurden über einen Backenbrecher vorzerkleinert und über eine Walzenmühle weiter gemahlen. Durch einen Siebschritt wurde die gewünschte Größenverteilung der Granulate durch die Wahl der entsprechenden Siebe erhalten.

c) Aktivierung des Katalysators

Die Aktivierung des Katalysators erfolgte in einer Standard-Glaslaborapparatur, z.B. einem Becherglas. Zu den Granulaten wurde unter Rühren eine wässrige Lauge (z.B. Natronlauge) gegeben. Die Granulate befanden sich während der Aktivierung in einem Katalysatorkorb. Durch den exothermen Vorgang der Aktivierung wird ein Teil des Aluminiums unter Bildung von Wasserstoff und Natriumaluminatlauge aus der Legierung herausgelöst. Die Konzentration der eingesetzten Lauge lag bei 20 Gew.-% und die Reaktionstemperatur bei 90°C. Der Grad der Aktivierung wurde über die Reaktionszeit bestimmt. Nach der Aktivierung wurde der Katalysator mit Wasser gewaschen und anschließend unter Wasser gelagert.
Der eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 75,9 Gew.-% |
| Aluminium: | 20,0 Gew.-% |
| Chrom: | 1,5 Gew.-% |
| Nickel: | 2,6 Gew.-% |

Es wurde eine Siebfraktion eingesetzt mit Partikelgrößen des Katalysators, also der Granulate, mit einer statistischen Verteilung zwischen 2,0 bis 5,0 Millimeter (mm), wobei bis zu 10 % der Partikel oberhalb der genannten Obergrenze und bis zu 10 % der Partikel unterhalb der genannten Untergrenze liegen können.

Beispiel 2: Hydrierung von TMN

Für die Hydrierung von TMN zu TMD wurde ein 2 L-Batchautoklav mit eingebautem Katalysatorkorb verwendet. Dieser wurde mit 150 ml des zu testenden Hydrierkatalysators gefüllt. Zur Konditionierung des Katalysators wurde der Reaktor inkl. eingebautem Katalysatorkorb mit 1 L reinem Ammoniak gefüllt und für 20 h bei ca. 20°C gerührt. Nach dem Ablassen der Lösung wurde 700 g Lösung bestehend aus 15 Gew.-% IPN in Ammoniak in den Reaktor gegeben und die Reaktionslösung über eine externe Beheizung auf Reaktionstemperatur aufgewärmt. Durch die Zugabe von Wasserstoff wurde der Startpunkt der Hydrierung definiert. Der Druck im Autoklaven wurde dabei auf 250 bar eingestellt und der Versuch bis zum jeweiligen Vollumsatz bezogen auf TMN gefahren.
Die Zusammensetzung des Endprodukts wurde mittels Gaschromatographie ermittelt.

Das eingesetzte Edukt TMN wies eine Reinheit von 96,6 % auf. Die in der Auswertung angegebene TMD-Selektivität wurde wie folgt berechnet:

$$Selektivität\ (TMD) = \frac{Ausbeute\ (TMD)}{Reinheit\ (TMN)} * 100\%$$

Die Ergebnisse der Versuchsreihe zur TMN-Hydrierung mit dem erfindungsgemäßen Katalysator wurden in Tabelle 1 zusammengefasst. Es wurde Vollumsatz bezüglich TMN erreicht.

Tabelle 1: Erfindungsgemäßer Katalysator:

| Reaktionsbedingungen | 80°C, 250 bar | 120°C, 250 bar |
|---|---|---|
| Reaktionszeit | 2 h | 1 h |
| TMD-Selektivität | 99,6 % | 97,7 % |

Als Vergleichskatalysator wurde ein kommerzieller, geträgerter Cobalt-Katalysator (tablettierter Formkörper mit Durchmesser 3 mm) eingesetzt. Die Ergebnisse der Versuchsreihe zur TMN-Hydrierung mit dem Vergleichskatalysator wurden in Tabelle 2 zusammengefasst. Es wurde Vollumsatz bezüglich TMN erreicht.

Tabelle 2: Vergleichskatalysator:

| Reaktionsbedingungen | 80°C, 250 bar | 120°C, 250 bar |
|---|---|---|
| Reaktionszeit | 4 h | 2 h |
| TMD-Selektivität | 99,2 % | 94,6 % |

Die Ergebnisse zeigen eine höhere Aktivität und Selektivität des erfindungsgemäßen Katalysators für die Hydrierung von TMN zur Herstellung von TMD.

## Patentansprüche

**1.** Verfahren zur Herstellung von Trimethylhexamethylendiamin durch Hydrierung von Trimethylhexamethylendinitril enthaltenden Gemischen in Gegenwart von mindestens Ammoniak und Wasserstoff und einem Katalysator, in An- oder Abwesenheit von Lösungsmittel,
wobei der Katalysator die folgenden Eigenschaften aufweist:

I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

und
II. Der Katalysator liegt in Form von unregelmäßigen Partikeln als Granulat vor und weist nach der Aktivierung Partikelgrößen von 1 bis 8 Millimeter (mm) auf.

**2.** Verfahren zur Herstellung von Trimethylhexamethylendiamin nach Anspruch 1, **dadurch gekennzeichnet, dass**

I. Der Katalysator nach der Aktivierung in seiner Gesamtheit die folgende Zusammensetzung aufweist in Gewichtsprozent (Gew.-%), wobei sich die Anteile zu 100 Gew.-% addieren, bezogen auf die enthaltenden Metalle:

| | |
|---|---|
| Cobalt: | 55 bis 90 Gew.-% |
| Aluminium: | 5 bis 44,5 Gew.-% |
| Chrom: | 0,5 bis 5 Gew.-% |

**3.** Verfahren zur Herstellung von Trimethylhexamethylendiamin nach Anspruch 1, **dadurch gekennzeichnet, dass**

I. Der Katalysator nach der Aktivierung in seiner Gesamtheit die folgende Zusammensetzung aufweist in Gewichtsprozent (Gew.-%), wobei sich die Anteile zu 100 Gew.-% addieren, bezogen auf die enthaltenden Metalle:

| | |
|---|---|
| Cobalt: | 55 bis 88 Gew.-% |
| Aluminium: | 5 bis 44,5 Gew.-% |
| Nickel: | 0,5 bis 7 Gew.-% |

**4.** Verfahren zur Herstellung von Trimethylhexamethylendiamin nach Anspruch 1, **dadurch gekennzeichnet, dass**

I. Der Katalysator nach der Aktivierung in seiner Gesamtheit die folgende Zusammensetzung aufweist in Gewichtsprozent (Gew.-%), wobei sich die Anteile zu 100 Gew.-% addieren, bezogen auf die enthaltenden Metalle:

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

**5.** Verfahren zur Herstellung von Trimethylhexamethylendiamin nach Anspruch 1, **dadurch gekennzeichnet, dass**

I. Der Katalysator nach der Aktivierung in seiner Gesamtheit die folgende Zusammensetzung aufweist in Gewichtsprozent (Gew.-%), wobei sich die Anteile zu 100 Gew.-% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

**6.** Verfahren zur Herstellung von Trimethylhexamethylendiamin nach mindestens einem der vorherigen Ansprüche 1-5, **dadurch gekennzeichnet, dass**
die Partikelgrößen des Katalysators, also die Granulat-Teilchen von 2,5 bis 6 Millimeter (mm) variieren,
oder
die Partikelgrößen des Katalysators, also die Granulat-Teilchen von 3 bis 7 Millimeter (mm) variieren,
oder
die Partikelgrößen des Katalysators, also die Granulat-Teilchen von 2 bis 5 Millimeter (mm) variieren.

**7.** Verfahren zur Herstellung von Trimethylhexamethylendiamin nach mindestens einem der vorherigen Ansprüche 1-6, **dadurch gekennzeichnet, dass** der Katalysator durch Siebung der hergestellten Granulate erhalten wird.

**8.** Verfahren zur Herstellung von Trimethylhexamethylendiamin nach Anspruch 7, **dadurch gekennzeichnet, dass** der Katalysator durch Siebung der hergestellten Granulate erhalten wird, und
die Partikelgrößen des Katalysators, also die Granulat-Teilchen mit einer statistischen Verteilung zwischen 2,5 bis 5,5 Millimeter (mm), liegen, oder
die Partikelgrößen des Katalysators, also die Granulat-Teilchen mit einer statistischen Verteilung zwischen 3,5 bis 6,5 Millimeter (mm), liegen, oder
die Partikelgrößen des Katalysators, also die Granulat-Teilchen mit einer statistischen Verteilung zwischen 2 bis 5 Millimeter (mm), liegen, oder
die Partikelgrößen des Katalysators, also de Granulat-Teilchen mit einer statistischen Verteilung zwischen 3 bis 7 Millimeter (mm),
und wobei bis zu 10 % der Partikel oberhalb der genannten Obergrenze und bis zu 10 % der Partikel unterhalb der genannten Untergrenze liegen können.

**9.** Verfahren zur Herstellung von Trimethylhexamethylendiamin nach Anspruch 1 oder nach mindestens einem der vorherigen Ansprüche 6-8, **dadurch gekennzeichnet, dass** der Katalysator nach der Aktivierung in seiner Gesamtheit die folgende Zusammensetzung aufweist in Gewichtsprozent (Gew.-%), wobei sich die Anteile zu 100 Gew.-

% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und mit
Partikelgrößen des Katalysators, also die Granulat-Teilchen mit einer statistischen Verteilung zwischen 2,5 bis 5,5 Millimeter (mm),
oder
Partikelgrößen des Katalysators, also die Granulat-Teilchen mit einer statistischen Verteilung zwischen 3,5 bis 6,5 Millimeter (mm),
oder
Partikelgrößen des Katalysators, also die Granulat-Teilchen mit einer statistischen Verteilung zwischen 2 bis 5 Millimeter (mm),
oder
Partikelgrößen des Katalysators, also die Granulat-Teilchen mit einer statistischen Verteilung zwischen 3 bis 7 Millimeter (mm),
wobei bis zu 10 % der Partikel oberhalb der genannten Obergrenze und bis zu 10 % der Partikel unterhalb der genannten Untergrenze liegen können.

**10.** Verfahren zur Herstellung von Trimethylhexamethylendiamin nach mindestens einem der vorherigen Ansprüche, **dadurch gekennzeichnet, dass** die Katalysatoren zusätzlich Dotiermetalle enthalten, bevorzugt ausgewählt aus Mo, Fe, Ag, V, Ga, In, Bi, Ti, Zr und Mn, sowie den seltenen Erden.

**11.** Verfahren zur Herstellung von Trimethylhexamethylendiamin nach mindestens einem der vorherigen Ansprüche, **dadurch gekennzeichnet, dass** Katalysatoren Modifizierungsmittel enthalten, bevorzugt Alkali- und Erdalkalimetalle bzw. deren Verbindungen, bevorzugt Mg- und Li-Verbindungen.

**12.** Verfahren nach mindestens einem der vorherigen Ansprüche,
**dadurch gekennzeichnet,**
**dass** batchweise oder kontinuierlich, oder einstufig oder mehrstufig verfahren wird.

**13.** Verfahren nach mindestens einem der vorherigen Ansprüche,
**dadurch gekennzeichnet,**
**dass** die Hydrierung kontinuierlich in Festbettreaktoren durchgeführt wird, die in Riesel- oder Sumpffahrweise betrieben werden.

**14.** Verfahren nach mindestens einem der vorherigen Ansprüche,
**dadurch gekennzeichnet,**
**dass** die Hydrierung bei Temperaturen zwischen 20 und 150°C, bevorzugt zwischen 40 und 130°C, und Drücken von 0,3 bis 50 MPa, bevorzugt 5 bis 30 MPa, durchgeführt wird.

**15.** Verwendung eines Katalysators zur Herstellung von Trimethylhexamethylendiamin durch Hydrierung von Trimethylhexamethylendinitril enthaltenden Gemischen in Gegenwart von mindestens Ammoniak und Wasserstoff, wobei der Katalysator die folgenden Eigenschaften aufweist:

I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

oder

| | |
|---|---|
| Cobalt: | 55 bis 90 Gew.-% |
| Aluminium: | 5 bis 44,5 Gew.-% |
| Chrom: | 0,5 bis 5 Gew.-% |

oder

| | |
|---|---|
| Cobalt: | 55 bis 88 Gew.-% |
| Aluminium: | 5 bis 44,5 Gew.-% |
| Nickel: | 0,5 bis 7 Gew.-% |

oder

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

oder

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und
II. Der Katalysator liegt in Form von unregelmäßigen Partikeln als Granulat vor und weist nach der Aktivierung Partikelgrößen von 1 bis 8 Millimeter (mm) auf.

## Claims

**1.** Process for producing trimethylhexamethylenediamine by hydrogenation of trimethylhexamethylenedinitrile-comprising mixtures in the presence of at least ammonia and hydrogen and a catalyst in the presence or absence of solvent,
wherein the catalyst has the following properties:

I. After activation the catalyst in its entirety has the following composition in weight percent (wt%), wherein the proportions add up to 100 wt%, based on the metals present:

| | |
|---|---|
| cobalt: | 55 to 95 wt% |
| aluminium: | 5 to 45 wt% |
| chromium: | 0 to 3 wt% |
| nickel: | 0 to 7 wt% |

and
II. The catalyst is present in the form of irregular particles as granulate and after activation has particle sizes of 1 to 8 millimetres (mm).

**2.** Process for producing trimethylhexamethylenediamine according to Claim 1, **characterized in that**

I. After activation the catalyst in its entirety has the following composition in weight percent (wt%), wherein the proportions add up to 100 wt%, based on the metals present:

| | |
|---|---|
| cobalt: | 55 to 90 wt% |
| aluminium: | 5 to 44.5 wt% |
| chromium: | 0.5 to 5 wt% |

3. Process for producing trimethylhexamethylenediamine according to Claim 1, **characterized in that**

I. After activation the catalyst in its entirety has the following composition in weight percent (wt%), wherein the proportions add up to 100 wt%, based on the metals present:

| | |
|---|---|
| cobalt: | 55 to 88 wt% |
| aluminium: | 5 to 44.5 wt% |
| nickel: | 0.5 to 7 wt% |

4. Process for producing trimethylhexamethylenediamine according to Claim 1, **characterized in that**

I. After activation the catalyst in its entirety has the following composition in weight percent (wt%), wherein the proportions add up to 100 wt%, based on the metals present:

| | |
|---|---|
| cobalt: | 55 to 85 wt% |
| aluminium: | 5 to 43.5 wt% |
| chromium: | 0.5 to 3 wt% |
| nickel: | 1 to 7 wt% |

5. Process for producing trimethylhexamethylenediamine according to Claim 1, **characterized in that**

I. After activation the catalyst in its entirety has the following composition in weight percent (wt%), wherein the proportions add up to 100 wt%, based on the metals present:

| | |
|---|---|
| cobalt: | 57 to 84 wt% |
| aluminium: | 10 to 40 wt% |
| chromium: | 1 to 2 wt% |
| nickel: | 2 to 4 wt% |

6. Process for producing trimethylhexamethylenediamine according to at least one of the preceding Claims 1-5, **characterized in that**
the particle sizes of the catalyst, i.e. the granulate particles, vary from 2.5 to 6 millimetres (mm),
or
the particle sizes of the catalyst, i.e. the granulate particles, vary from 3 to 7 millimetres (mm),
or
the particle sizes of the catalyst, i.e. the granulate particles, vary from 2 to 5 millimetres (mm).

7. Process for producing trimethylhexamethylenediamine according to at least one of the preceding Claims 1-6, **characterized in that** the catalyst is obtained by sieving the granulates produced.

8. Process for preparing trimethylhexamethylenediamine according to Claim 7, **characterized in that** the catalyst is obtained by sieving the granulates produced, and
the particle sizes of the catalyst, i.e. the granulate particles, have a statistical distribution between 2.5 to 5.5 millimetres (mm), or
the particle sizes of the catalyst, i.e. the granulate particles, have a statistical distribution between 3.5 to 6.5 millimetres (mm), or

the particle sizes of the catalyst, i.e. the granulate particles, have a statistical distribution between 2 to 5 millimetres (mm), or
the particle sizes of the catalyst, i.e. the granulate particles, have a statistical distribution between 3 to 7 millimetres (mm),
and wherein up to 10% of the particles may be above the upper limit mentioned and up to 10% of the particles may be below the lower limit mentioned.

**9.** Process for preparing trimethylhexamethylenediamine according to Claim 1 or according to at least one of the preceding Claims 6-8, **characterized in that** after activation the catalyst in its entirety has the following composition in weight percent (wt%), wherein the proportions add up to 100 wt%, based on the metals present:

| | |
|---|---|
| cobalt: | 57 to 84 wt% |
| aluminium: | 10 to 40 wt% |
| chromium: | 1 to 2 wt% |
| nickel: | 2 to 4 wt% |

and with
particle sizes of the catalyst, i.e. the granulate particles, having a statistical distribution between 2.5 to 5.5 millimetres (mm),
or
particle sizes of the catalyst, i.e. the granulate particles, having a statistical distribution between 3.5 to 6.5 millimetres (mm),
or
particle sizes of the catalyst, i.e. the granulate particles, having a statistical distribution between 2 to 5 millimetres (mm),
or
particle sizes of the catalyst, i.e. the granulate particles, having a statistical distribution between 3 to 7 millimetres (mm),
wherein up to 10% of the particles may be above the upper limit mentioned and up to 10% of the particles may be below the lower limit mentioned.

**10.** Process for preparing trimethylhexamethylenediamine according to at least one of the preceding claims, **characterized in that** the catalysts additionally comprise doping metals, preferably selected from Mo, Fe, Ag, V, Ga, In, Bi, Ti, Zr and Mn and also the rare earths.

**11.** Process for preparing trimethylhexamethylenediamine according to at least one of the preceding claims, **characterized in that** catalysts comprise modifiers, preferably alkali metals and alkaline earth metals or compounds thereof, preferably magnesium and lithium compounds.

**12.** Process according to at least one of the preceding claims,
**characterized in that**
it is a batchwise or continuous, single-stage or multi-stage process.

**13.** Process according to at least one of the preceding claims,
**characterized in that**
the hydrogenation is performed continuously in fixed bed reactors which are operated in downflow or upflow mode.

**14.** Process according to at least one of the preceding claims,
**characterized in that**
the hydrogenation is performed at temperatures between 20°C and 150°C, preferably between 40°C and 130°C, and pressures of 0.3 to 50 MPa, preferably 5 to 30 MPa.

**15.** Use of a catalyst for producing trimethylhexamethylenediamine by hydrogenation of trimethylhexamethylenedinitrile-comprising mixtures in the presence of at least ammonia and hydrogen, wherein the catalyst has the following properties:

I. After activation the catalyst in its entirety has the following composition in weight percent (wt%), wherein the

proportions add up to 100 wt%, based on the metals present:

| | |
|---|---|
| cobalt: | 55 to 95 wt% |
| aluminium: | 5 to 45 wt% |
| chromium: | 0 to 3 wt% |
| nickel: | 0 to 7 wt% |

or

| | |
|---|---|
| cobalt: | 55 to 90 wt% |
| aluminium: | 5 to 44.5 wt% |
| chromium: | 0.5 to 5 wt% |

or

| | |
|---|---|
| cobalt: | 55 to 88 wt% |
| aluminium: | 5 to 44.5 wt% |
| nickel: | 0.5 to 7 wt% |

or

| | |
|---|---|
| cobalt: | 55 to 85 wt% |
| aluminium: | 5 to 43.5 wt% |
| chromium: | 0.5 to 3 wt% |
| nickel: | 1 to 7 wt% |

or

| | |
|---|---|
| cobalt: | 57 to 84 wt% |
| aluminium: | 10 to 40 wt% |
| chromium: | 1 to 2 wt% |
| nickel: | 2 to 4 wt% |

and
II. The catalyst is present in the form of irregular particles as granulate and after activation has particle sizes of 1 to 8 millimetres (mm).

## Revendications

**1.** Procédé de fabrication de triméthylhexaméthylène-diamine par hydrogénation de mélanges contenant du triméthylhexaméthylène-dinitrile en présence au moins d'ammoniac et d'hydrogène et d'un catalyseur, en présence ou en absence de solvants,
le catalyseur présentant les propriétés suivantes :

I. le catalyseur présente dans son ensemble après l'activation la composition suivante en pourcentages en poids (% en poids), la somme des proportions étant de 100 % en poids, au regard des métaux contenus :

cobalt : 55 à 95 % en poids,
aluminium : 5 à 45 % en poids,
chrome : 0 à 3 % en poids,
nickel : 0 à 7 % en poids,
et

II. le catalyseur se présente sous la forme de particules irrégulières sous la forme d'un granulat et présente après l'activation des tailles de particules de 1 à 8 millimètres (mm).

**2.** Procédé de fabrication de triméthylhexaméthylène-diamine selon la revendication 1, **caractérisé en ce que**

I. le catalyseur présente dans son ensemble après l'activation la composition suivante en pourcentages en poids (% en poids), la somme des proportions étant de 100 % en poids, au regard des métaux contenus :

cobalt : 55 à 90 % en poids,
aluminium : 5 à 44,5 % en poids,
chrome : 0,5 à 5 % en poids.

**3.** Procédé de fabrication de triméthylhexaméthylène-diamine selon la revendication 1, **caractérisé en ce que**

I. le catalyseur présente dans son ensemble après l'activation la composition suivante en pourcentages en poids (% en poids), la somme des proportions étant de 100 % en poids, au regard des métaux contenus :

cobalt : 55 à 88 % en poids,
aluminium : 5 à 44,5 % en poids,
nickel : 0,5 à 7 % en poids.

**4.** Procédé de fabrication de triméthylhexaméthylène-diamine selon la revendication 1, **caractérisé en ce que**

I. le catalyseur présente dans son ensemble après l'activation la composition suivante en pourcentages en poids (% en poids), la somme des proportions étant de 100 % en poids, au regard des métaux contenus :

cobalt : 55 à 85 % en poids,
aluminium : 5 à 43,5 % en poids,
chrome : 0,5 à 3 % en poids,
nickel : 1 à 7 % en poids.

**5.** Procédé de fabrication de triméthylhexaméthylène-diamine selon la revendication 1, **caractérisé en ce que**

I. le catalyseur présente dans son ensemble après l'activation la composition suivante en pourcentages en poids (% en poids), la somme des proportions étant de 100 % en poids, au regard des métaux contenus :

cobalt : 57 à 84 % en poids,
aluminium : 10 à 40 % en poids,
chrome : 1 à 2 % en poids,
nickel : 2 à 4 % en poids.

**6.** Procédé de fabrication de triméthylhexaméthylène-diamine selon au moins l'une quelconque des revendications 1 à 5 précédentes, **caractérisé en ce que** les tailles de particules du catalyseur, c'est-à-dire les particules du granulat, varient de 2,5 à 6 millimètres (mm),
ou
les tailles de particules du catalyseur, c'est-à-dire les particules du granulat, varient de 3 à 7 millimètres (mm),
ou
les tailles de particules du catalyseur, c'est-à-dire les particules du granulat, varient de 2 à 5 millimètres (mm) .

**7.** Procédé de fabrication de triméthylhexaméthylène-diamine selon au moins l'une quelconque des revendications 1 à 6 précédentes, **caractérisé en ce que** le catalyseur est obtenu par tamisage des granulats fabriqués.

**8.** Procédé de fabrication de triméthylhexaméthylène-diamine selon la revendication 7, **caractérisé en ce que** le catalyseur est obtenu par tamisage des granulats fabriqués, et
les tailles de particules du catalyseur, c'est-à-dire les particules du granulat, présentent une distribution statistique comprise entre 2,5 et 5,5 millimètres (mm), ou
les tailles de particules du catalyseur, c'est-à-dire les particules du granulat, présentent une distribution statistique comprise entre 3,5 et 6,5 millimètres (mm), ou

les tailles de particules du catalyseur, c'est-à-dire les particules du granulat, présentent une distribution statistique comprise entre 2 et 5 millimètres (mm), ou les tailles de particules du catalyseur, c'est-à-dire les particules du granulat, présentent une distribution statistique comprise entre 3 et 7 millimètres (mm),
et jusqu'à 10 % des particules peuvent se situer au-dessus de la limite supérieure indiquée et jusqu'à 10 % des particules peuvent se situer en dessous de la limite inférieure indiquée.

**9.** Procédé de fabrication de triméthylhexaméthylène-diamine selon la revendication 1 ou selon au moins l'une quelconque des revendications 6 à 8 précédentes, **caractérisé en ce que** le catalyseur présente dans son ensemble après l'activation la composition suivante en pourcentages en poids (% en poids), la somme des proportions étant de 100 % en poids, au regard des métaux contenus :

cobalt : 57 à 84 % en poids,
aluminium : 10 à 40 % en poids,
chrome : 1 à 2 % en poids,
nickel : 2 à 4 % en poids,
et présente
des tailles de particules du catalyseur, c'est-à-dire des particules du granulat, ayant une distribution statistique comprise entre 2,5 et 5,5 millimètres (mm),
ou
des tailles de particules du catalyseur, c'est-à-dire des particules du granulat, ayant une distribution statistique comprise entre 3,5 et 6,5 millimètres (mm),
ou
des tailles de particules du catalyseur, c'est-à-dire des particules du granulat, ayant une distribution statistique comprise entre 2 et 5 millimètres (mm),
ou
des tailles de particules du catalyseur, c'est-à-dire des particules du granulat, ayant une distribution statistique comprise entre 3 et 7 millimètres (mm),
jusqu'à 10 % des particules pouvant se situer au-dessus de la limite supérieure indiquée et jusqu'à 10 % des particules pouvant se situer en dessous de la limite inférieure indiquée.

**10.** Procédé de fabrication de triméthylhexaméthylène-diamine selon au moins l'une quelconque des revendications précédentes, **caractérisé en ce que** les catalyseurs contiennent en outre des métaux dopants, de préférence choisis parmi Mo, Fe, Ag, V, Ga, In, Bi, Ti, Zr et Mn, ainsi que les terres rares.

**11.** Procédé de fabrication de triméthylhexaméthylène-diamine selon au moins l'une quelconque des revendications précédentes, **caractérisé en ce que** les catalyseurs contiennent des agents de modification, de préférence des métaux alcalins et alcalino-terreux ou leurs composés, de préférence des composés de Mg et Li.

**12.** Procédé selon au moins l'une quelconque des revendications précédentes, **caractérisé en ce qu'**il est réalisé de manière discontinue ou continue, ou en une étape ou en plusieurs étapes.

**13.** Procédé selon au moins l'une quelconque des revendications précédentes, **caractérisé en ce que** l'hydrogénation est réalisée en continu dans des réacteurs à lit fixe, qui sont exploités en mode de ruissellement ou de fond.

**14.** Procédé selon au moins l'une quelconque des revendications précédentes, **caractérisé en ce que** l'hydrogénation est réalisée à des températures comprises entre 20 et 150 °C, de préférence entre 40 et 130 °C, et des pressions de 0,3 à 50 MPa, de préférence de 5 à 30 MPa.

**15.** Utilisation d'un catalyseur pour la fabrication de triméthylhexaméthylène-diamine par hydrogénation de mélanges contenant du triméthylhexaméthylène-dinitrile en présence au moins d'ammoniac et d'hydrogène,
le catalyseur présentant les propriétés suivantes :

I. le catalyseur présente dans son ensemble après l'activation la composition suivante en pourcentages en poids (% en poids), la somme des proportions étant de 100 % en poids, au regard des métaux contenus :

cobalt : 55 à 95 % en poids,
aluminium : 5 à 45 % en poids,
chrome : 0 à 3 % en poids,

nickel : 0 à 7 % en poids,
ou
cobalt : 55 à 90 % en poids,
aluminium : 5 à 44,5 % en poids,
chrome : 0,5 à 5 % en poids,
ou
cobalt : 55 à 88 % en poids,
aluminium : 5 à 44,5 % en poids,
nickel : 0,5 à 7 % en poids,
ou
cobalt : 55 à 85 % en poids,
aluminium : 5 à 43,5 % en poids,
chrome : 0,5 à 3 % en poids,
nickel : 1 à 7 % en poids,
ou
cobalt : 57 à 84 % en poids,
aluminium : 10 à 40 % en poids,
chrome : 1 à 2 % en poids,
nickel : 2 à 4 % en poids,
et

II. le catalyseur se présente sous la forme de particules irrégulières sous la forme d’un granulat et présente après l’activation des tailles de particules de 1 à 8 millimètres (mm).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4345265
- DE 4335360
- EP 880996 A
- EP 2114859 A


### In der Beschreibung aufgeführte Nicht-Patentliteratur


- **PAUL SCHMIDT ; ROLF KÖRBER ; MATTHIAS COPPERS.** Sieben und Siebmaschinen: Grundlagen und Anwendung. Wiley-VCH Verlag, 2003
- **JÖRG HOFFMANN.** Handbuch der Messtechnik. Hanser Verlag, 2007